(19) Europäisches Patentamt
European Patent Office
Office européen des brevets

(11) Publication number: **0 558 563 B1**

(12) **EUROPEAN PATENT SPECIFICATION**

(45) Date of publication of patent specification: **29.03.95**

(51) Int. Cl.[6]: **C07C 275/64**, C07D 333/58, A61K 31/17, A61K 31/38

(21) Application number: **91920193.9**

(22) Date of filing: **22.11.91**

(86) International application number: **PCT/GB91/02067**

(87) International publication number: **WO 92/09567 (11.06.92 92/13)**

# (54) ANTI-INFLAMMATORY COMPOUNDS.

(30) Priority: **23.11.90 GB 9025514**

(43) Date of publication of application: **08.09.93 Bulletin 93/36**

(45) Publication of the grant of the patent: **29.03.95 Bulletin 95/13**

(84) Designated Contracting States: **AT BE CH DE DK ES FR GB GR IT LI LU NL SE**

(56) References cited:
**EP-A- 0 436 199**

**Journal of Medicinal Chemistry, volume 25, no. 10, 1982, American Chemical Society, Washington, US; A.A. Elfarra et al.: "Synthesis and evaluation of N-(phenylalkyl)acetohydroxamic acids as potential substrates for N-arylhydroxamic acid N,O-acyltransferase", pages 1189-1192, see table 1, compound 6; page 1190, scheme III**

(73) Proprietor: **THE WELLCOME FOUNDATION LIMITED**
**Unicorn House**
**160 Euston Road**
**London NW1 2BP (GB)**

(72) Inventor: **HODGSON, Simon, Teanby Langley Court**
**Beckenham**
**Kent BR3 3BS (GB)**
Inventor: **WATES, Peter, John Langley Court**
**Beckenham**
**Kent BR3 3BS (GB)**
Inventor: **DAVIES, David, Evan Langley Court**
**Beckenham**
**Kent BR3 3BS (GB)**
Inventor: **SMITH, Steven**
**Langley Court**
**Beckenham**
**Kent BR3 3BS (GB)**
Inventor: **DEMAINE, Derek, Anthony**
**Langley Court**
**Beckenham**
**Kent BR3 3BS (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.09/3.3.3)

(74) Representative: **Rollins, Anthony John et al**
**Group Patents & Agreements**
**The Wellcome Foundation Ltd**
**Langley Court**
**Beckenham**
**Kent BR3 3BS (GB)**

## Description

The present invention is concerned with a genus of novel hydroxamic acid derivatives having anti-inflammatory activity, with processes for their preparation, with pharmaceutical formulations containing said derivatives and with their use in medicine.

European Patent Specification (EPS) 0196184 describes a genus of hydroxamic acid derivatives having anti-inflammatory activity by virtue of their ability to inhibit the enzyme 5-lipoxygenase in the mammalian arachidonic acid cascade. EPS 0299761 directed to a sub-genus of hydroxamates having prolonged duration of action and EPS 0384594 concerned with a further sub-genus of hydroxyureas having similar properties both relate to selection inventions derived from EPS 0196184. Unfortunately, the compounds of EPS 0299761 and EPS 0384594 both contain chiral centres which give rise to complications associated with the different ways in which enantiomers can be metabolised in vivo, the difficulties of synthesising enantiomerically pure compounds and the increasing reluctance of drug regulatory authorities to approve racemic mixtures.

US 5,037,853 describes certain aryl-substituted cyclopropyl N-hydroxyureas possessing 5-lipoxygenase inhibitory activity, but having a chiral centre.

We have now identified a genus of hydroxamic acid derivatives wherein the presence of a cycloalkyl moiety can serve to eliminate the chiral centre associated with the compounds of EPS 0299761, EPS 0384594 and US 5,037,853, while retaining the desirable pharmacological properties of the earlier compounds. Even those compounds of the present invention which have a chiral centre possess highly desirable pharmacological properties.

According to the present application, therefore, there is provided a compound of formula (I)

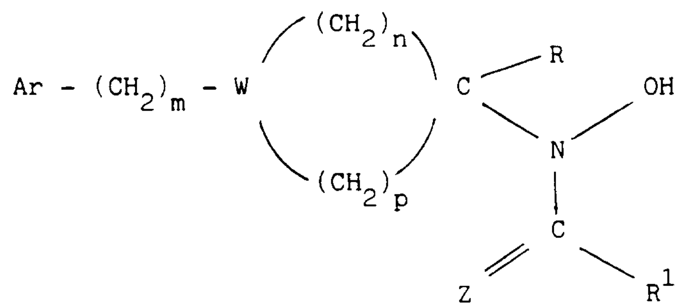

(I)

wherein

- m is O or 1;
- n is an integer of from 1 to 6 and p is an integer of from 0 to 3 with the proviso that $n + p$ does not exceed 6;
- Ar is a mono-, bi-, or tricyclic aromatic ring system wherein one or more of the carbon atoms is optionally replaced by a heteroatom(s) independently selected from oxygen, sulphur and nitrogen, which ring system is optionally substituted by one or more atoms or groups independently selected from (i) $C_{1-4}$ alkyl (wherein one or more of the hydrogen atoms may be replaced by halogen), $C_{1-4}$ alkoxy, halogen, nitro, amino, carboxy, $C_{2-5}$ alkoxycarbonyl, hydroxy, cyano, carbamoyl and alkylsulphonyl, and (ii) phenoxy, benzyloxy and naphthyloxy, which aryloxy groups are optionally substituted by one or more substituents independently selected from those specified in (i);
- R is hydrogen, $C_{1-6}$ alkyl, vinyl, $C_{1-6}$ alkoxy($C_{1-6}$ alkyl), or carbamoyl;
- $R^1$ is $C_{1-6}$ alkyl (including $C_{3-6}$ cycloalkyl), phenyl, or benzyl, which phenyl or benzyl group is optionally substituted by one or more substituents independently selected from those specified in (i), or $-NR^2R^3$ wherein $R^2$ and $R^3$ are independently selected from hydrogen, $C_{1-6}$ alkyl (including $C_{3-6}$ cycloalkyl), phenyl and benzyl, which phenyl and benzyl groups are optionally substituted by one or more substituents independently selected from those specified in (i);
- W is -N< or $-C(R^4)$< where $R^4$ is hydrogen, $C_{1-4}$ alkyl, or phenyl; and
- Z is oxygen or sulphur;

with the provisoes that

(i) $n + p \neq 1$ when W is $-C(R^4)$; or

(ii) n + p ≠ 4 when Ar is an unsubstituted phenyl group;

and base salts and physiologically functional derivatives thereof.

Preferred compounds of formula (I) include those wherein

m is 0;

n = p = 1 or 2;

Ar is a substituted phenyl or an optionally substituted benzothienyl group, 4-fluorophenoxy being a particularly preferred substituent;

R is hydrogen;

$R^1$ is $C_{1-4}$ alkyl or $-NR^2R^3$ wherein $R^2$ and $R^3$ are both hydrogen;

W is -CH< ; and

Z is oxygen;

and base salts and physiologically functional derivatives thereof.

It will be appreciated that the compounds of formula (I) and their base salts may exist as either the cis or trans geometric isomer according to the relative positions of the cycloalkyl substituents above and below the plane of the ring. The present invention includes within its scope each of these isomers as well as mixtures thereof in any proportions.

Particularly preferred compounds of formula (I) having exceptional 5-lipoxygenase inhibitory properties are cis-1-{3-(3-(4-fluorophenoxy)phenyl]cyclobutyl}-1-hydroxyurea, trans-1-hydroxy-1-[3-(2-benzo[b]-thienyl)cyclobutyl]urea and their base salts and physiologically functional derivatives.

Base salts of compounds of formula (I) which are suitable for use in medicine are those wherein the cation is physiologically acceptable. However, base salts having non-physiologically acceptable cations are within the ambit of the present invention, either for use in non-medical applications or as intermediates in the preparation of compounds of formula (I) and their physiologically acceptable base salts and physiologically functional derivatives.

Salts according to the invention include ammonium salts, alkali metal salts such as those of sodium and potassium, alkaline earth metal salts such as those of calcium and magnesium, salts with organic bases such as dicyclohexylamine and N-methyl-D-glucamine, and salts with amino acids, such as arginine and lysine.

The above compounds of formula (I) and their physiologically acceptable base salts and physiologically functional derivatives are hereinafter referred to as "compounds of the invention" in relation to the therapeutic and pharmaceutical aspects of the invention discussed below.

According to further aspects of the invention, there are provided:

(a) compounds of the invention for use in therapy;

(b) pharmaceutical formulations containing a compound of the invention, at least one pharmaceutical carrier or excipient and, optionally, one or more other therapeutic ingredients;

(c) the use of a compound of the invention in the manufacture of a medicament for the prophylaxis or treatment of a clinical condition for which a 5-lipoxygenase inhibitor is indicated;

(d) the use of a compound of the invention in the manufacture of a medicament for the prophylaxis or treatment of

- (i) a spasmogenic condition,
- (ii) an allergic condition,
- (iii) tumour formation,
- (iv) a condition involving blood platelet aggregation,
- (v) an inflammatory condition;

(e) processes for the preparation of the compounds of the invention.

By virtue of their 5-lipoxygenase inhibitory properties, the compounds of the invention find application in the prophylaxis or treatment of clinical conditions for which an inhibitor of the lipoxygenase-mediated arachidonic acid metabolic pathway is indicated, especially

(i) spasmogenic conditions,

(ii) allergic conditions,

(iii) tumour formation,

(iv) conditions involving blood platelet aggregation, and

(v) inflammatory conditions.

These will be discussed in turn.

(i) Examples of spasmogenic conditions are those involving smooth muscle tissue, especially airway smooth muscle constriction such as intrinsic asthma (including idiopathic bronchial asthma and cardiac asthma), bronchitis and arterial smooth muscle constriction such as coronary spasm (including that associated with myocardial infarction, which may or may not lead to left ventricular failure resulting in

cardiac asthma) and cerebral spasm or 'stroke'. Other examples include bowel disease caused by abnormal colonic muscular contraction such as the conditions known as 'irritable bowel syndrome', 'spastic colon' and 'mucous colitis'.

(ii) Examples of allergic conditions are extrinsic asthma, allergic skin diseases having a total or partial allergic origin, such as eczema, allergic bowel diseases (including coeliac disease), allergic eye conditions, such as hayfever (which may additionally or alternatively affect the upper respiratory tract), and allergic conjunctivitis.

(iii) Examples of tumours are skin neoplasms, mastocytoma and other forms of cellular proliferation, both benign and malignant. It is to be noted that the effectiveness of the present compounds in the prophylaxis and treatment of tumours may arise from properties in addition to 5-lipoxygenase inhibition which also inhibit cell proliferation.

(iv) Examples of conditions involving blood platelet aggregation are those resulting from thrombosis, including 'strokes' having a total or partial thrombotic origin, coronary thrombosis, phlebitis and phlebothrombosis (the latter two conditions also possibly being associated with inflammation).

(v) Examples of inflammatory conditions are those of the (a) lungs, (b) joints, (c) eyes, (d) bowel, (e) skin and (f) heart, particularly those associated with the infiltration of leucocytes into inflamed tissue. Examples of such inflammatory conditions are:

(a) Inflammatory lung conditions include asthma, bronchitis and cystic fibrosis (which may additionally or alternatively involve the bowel or other tissue(s)).

(b) Inflammatory joint conditions include rheumatoid arthritis, rheumatoid spondylitis, osteoarthritis, gouty arthritis and other arthritic conditions.

(c) Inflammatory eye conditions include uveitis (including iritis) and conjunctivitis.

(d) Inflammatory bowel conditions include Crohn's disease, ulcerative colitis and distal proctitis.

(e) Inflammatory skin diseases include those associated with cell proliferation, such as psoriasis, eczema and dermatitis (whether or not of allergic origin).

(f) Inflammatory conditions of the heart include coronary infarct damage.

Other inflammatory conditions include tissue necrosis in chronic inflammation and tissue rejection following transplant surgery.

In view of their unique properties the compounds of the invention may also be employed in the prophylaxis or treatment of smooth muscle proliferation disorders, for example, restenosis following angioplasty, bacterial and fungal infections, dysmenorrhoea, multiple sclerosis and clinical conditions for which an immunosuppressant, anti-convulsant, or analgesic is indicated. The compounds of the invention also exhibit hypocholesterolaemic activity.

The amount required of the compound of the invention (hereinafter referred to as the active ingredient) to achieve a therapeutic effect will, of course, vary with the particular compound, the route of administration, the subject under treatment, and the particular disorder or disease being treated. A suitable dose for a mammal suffering from, or likely to suffer from, any of the clinical conditions described hereinbefore is in the range 0.1μg to 500mg of compound/kilogram bodyweight. In the case of systemic administration, the dose is typically in the range 0.5 to 500mg of compound/kilogram bodyweight, the most preferred dosage being 0.5 to 50mg/kg bodyweight, for example, 5 to 25mg/kg, administered two or three times daily. In the case of topical administration, e.g. to the skin or eye, a suitable dose is in the range 0.1ng - 100μg of base per kilogram, typically about 0.1 μg/kg.

In the case of oral dosing for the prophylaxis or treatment of airway smooth muscle constriction, for example, in asthma or bronchitis, a suitable dose of the compound of the invention may be as specified in the preceding paragraph, but most preferably is from 1mg to 10mg of base per kilogram bodyweight, the most preferred dosage being from 1mg to 5mg/kg bodyweight, for example, from 1 to 2 mg/kg. In the case of pulmonary administration, the dose is typically in the range 2μg to 100mg/kg, for example, from 20μg to 0.5mg/kg, especially from 0.1 to 0.5 mg/kg.

While it is possible for the active ingredient to be administered alone, it is preferable to present it as a pharmaceutical formulation comprising a compound of the invention in association with a pharmaceutically acceptable carrier or excipient and, optionally, one or more other therapeutic ingredients. The carrier or excipient must, of course, be compatible with the other ingredients in the formulation and must not be detrimental to the recipient. The active ingredient may comprise from 0.1% to 99.9% by weight of the formulation. Typical unit doses of a formulation according to the invention contain from 0.1mg to 1g of the active ingredient. For topical administration, the active ingredient preferably constitutes from 1% to 2% by weight of the formulation, but the active ingredient may constitute as much as 10% w/w. Formulations suitable for nasal or buccal administration, typically contain from 0.1 to 20% w/w, for example, 2% w/w of the active ingredient.

Formulations according to the invention include those in a form suitable for oral, pulmonary, ophthalmic, rectal, parenteral (including subcutaneous, intramuscular and intravenous), intra-articular, topical, or nasal/buccal administration.

The formulations of the invention may conveniently be presented in unit dosage form and may be prepared by any method well known in the art of pharmacy. All such methods include the step of bringing the active ingredient into association with a carrier which constitutes one or more accessory ingredients. In general, the formulations are prepared by uniformly and intimately bringing the active ingredient into association with a liquid carrier or a finely divided solid carrier, or both, and then, if desired, shaping the product into the required form.

Formulations according to the present invention which are suitable for oral administration may be in the form of discrete units such as capsules, cachets, tablets, or lozenges, each containing a predetermined amount of the active ingredient; in the form of a powder or granules; in the form of a solution or a suspension in an aqueous or non-aqueous liquid; or in the form of an oil-in-water or water-in-oil emulsion. The active ingredient may also be in the form of a bolus, electuary, or paste.

A tablet may be made by compressing or moulding the active ingredient, optionally with one or more accessory ingredients. Compressed tablets may be prepared by compressing, in a suitable machine, the active ingredient in a free-flowing form such as a powder or granules, optionally mixed with a binder, lubricant, inert diluent, and/or surface active or dispersing agent. Moulded tablets may be made by moulding, in a suitable machine, a mixture of the powdered active ingredient and a suitable carrier moistened with an inert liquid diluent.

Formulations for rectal administration may be in the form of a suppository incorporating the active ingredient and a carrier such as cocoa butter, or in the form of an enema.

Formulations suitable for parenteral administration typically comprise a sterile aqueous preparation of the active ingredient which is preferably isotonic with the blood of the recipient.

Formulations suitable for intra-articular administration may be in the form of a sterile aqueous preparation of the active ingredient, which latter may be in microcrystalline form, for example, an aqueous microcrystalline suspension. Liposomal formulations and biodegradable polymer systems may also be used to present the active ingredient for both intra-articular and ophthalmic administration.

Formulations suitable for topical administration include liquid and semi-liquid preparations such as liniments, lotions and applications; oil-in-water and water-in-oil emulsions such as creams, ointments and pastes; and solutions and suspensions such as drops. For example, for ophthalmic administration, the active ingredient may be presented as aqueous eye drops, for example, in the form of a 0.1 - 1.0% w/v solution.

Suitable formulations for administration by inhalation include fine particle dusts or mists which may be generated by means of various types of metered dose pressurised aerosols, nebulisers, or insufflators.

For pulmonary administration via the mouth, the particle size of the powder or droplets is typically in the range 0.5 - 10μm, preferably 1 - 5μm, to ensure delivery into the bronchial tree. For nasal administration, a particle size in the range 10 - 500μm is preferred to ensure retention in the nasal cavity.

Metered dose inhalers are pressurised aerosol dispensers, typically containing a suspension or solution formulation of the active ingredient in a liquefied propellant. During use these devices discharge the formulation through a valve adapted to deliver a metered volume, typically from 10 to 150μl, to produce a fine particle spray containing the active ingredient. Suitable propellants include certain chlorofluorocarbon compounds, for example, dichlorodifluoromethane, trichlorofluoromethane, dichlorotetrafluoroethane and mixtures thereof. The formulation may additionally contain one or more co-solvents, for example, ethanol, surfactants, such as oleic acid or sorbitan trioleate, antioxidants and suitable flavouring agents.

Nebulisers are commercially available devices that transform solutions or suspensions of the active ingredient into a therapeutic aerosol mist either by means of acceleration of a compressed gas through a narrow venturi orifice, typically air or oxygen, or by means of ultrasonic agitation. Suitable formulations for use in nebulisers consist of the active ingredient in a liquid carrier and comprising up to 40% w/w of the formulation, preferably less than 20% w/w. The carrier is typically water or a dilute aqueous alcoholic solution, preferably made isotonic with body fluids by the addition of, for example, sodium chloride. Optional additives include preservatives if the formulation is not prepared sterile, for example, methyl hydroxybenzoate, antioxidants, flavouring agents, volatile oils, buffering agents and surfactants.

Suitable formulations for administration by insufflation include finely comminuted powders which may be delivered by means of an insufflator or taken into the nasal cavity in the manner of a snuff. In the insufflator, the powder is contained in capsules or cartridges, typically made of gelatin or plastic, which are either pierced or opened in situ and the powder delivered by air drawn through the device upon inhalation or by means of a manually-operated pump. The powder employed in the insufflator consists either solely of the active ingredient or of a powder blend comprising the active ingredient, a suitable powder diluent, such

as lactose, and an optional surfactant. The active ingredient typically comprises from 0.1 to 100 w/w of the formulation.

In addition to the aforementioned ingredients, formulations according to the invention may include one or more additional ingredients such as diluents, buffers, flavouring agents, binders, surface active agents, thickeners, lubricants, preservatives, for example, methyl hydroxybenzoate, anti-oxidants and emulsifying agents.

The compounds of the invention may advantageously be employed in combination with one or more other therapeutic ingredients selected from an antibiotic (for example, an anti-bacterial), anti-fungal, or anti-viral agent, an anti-histamine (particularly a peripherally-acting anti-histamine), Or a non-steroidal anti-inflammatory drug (NSAID). The therapeutic components Of the combination may be administered simultaneously, either in the same formulation or in separate formulations, or sequentially within a sufficiently short time interval to achieve the desired combined therapeutic effect.

According to a further aspect of the invention, there is provided a process for preparing a compound of formula (I) wherein m, n, p, Ar, R, $R^1$, W and Z are as hereinbefore defined, which comprises reacting a compound of formula (II)

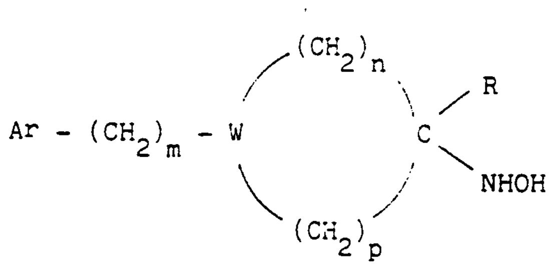

(II)

wherein m, n, p, Ar, R and W are as hereinbefore defined, with a suitable agent or agents and under such reaction conditions as to effect conversion of the N-hydrogen to an N-C(Z)$R^1$ group. When $R^1$ is to be amino the reaction is typically carried out by treatment of the compound of formula (II) with, in the case where Z is to be oxygen, a Group I cyanate, for example, potassium cyanate, in the presence of acid, or, in the case where Z is to be sulphur, trimethylsilylisothiocyanate, in a non-polar solvent, such as tetrahydrofuran (THF).

Hydroxylamines of formula (II) may be prepared by acid hydrolysis of the corresponding -N(BOC)OBOC compound where BOC is *t*-butoxycarbonyl. The *bis*-BOC compound may be prepared by treating the corresponding cyclic alcohol with HN(BOC)OBOC *in* the presence of triphenylphosphine/diethyl azodicarboxylate (DEAD) at low temperature in a non-polar solvent such as THF. The cyclic alcohol may be prepared from the corresponding cyclic ketone by reduction using, for example, lithium aluminium hydride in a non-polar solvent, such as THF, or, in the case where R is to be other than hydrogen, by a Grignard reaction.

In the case where n = p = 1 and W is $-C(R^4)<$ as hereinbefore defined, the appropriate cyclobutanone may be prepared by dehalogenation of the corresponding $\alpha,\alpha$-dihalocyclobutanone using, for example, activated zinc powder in glacial acetic acid. The dihalocyclobutanone may be prepared by $2\pi + 2\pi$ addition of the corresponding alkene to "2,2-dihaloketene" prepared *in situ*, typically using trihaloacetyl chloride and phosphoryl chloride in the presence of activated zinc, in a non-polar solvent, such as ether. The alkene may be prepared from the corresponding aldehyde by a Wittig reaction using, for example, methyltriphenylphosphonium bromide and potassium *t*-butoxide in a non-polar solvent, such as THF. The aldehyde may be obtained commercially or prepared by synthetic methods well known to a skilled man.

In the case where n = p = 2 and W is $-C(R^4)<$ as hereinbefore defined, the appropriate cyclohexanone may be prepared by acid hydrolysis of the corresponding acetal, typically using trifluoroacetic acid. The acetal may be prepared by catalytic reduction of the corresponding cyclohexene, typically by hydrogenation over Pd/C. The cyclohexene may be prepared by dehydration of the corresponding cyclohexanol, typically using thionyl chloride in pyridine. The cyclohexanol may be prepared by reductive arylation of the corresponding cyclohexanone, typically using $Ar-(CH_2)_m-Li$, where m and Ar are as hereinbefore defined, in a non-polar solvent, such as THF. The cyclohexanone may be prepared by monoacetalisation of the corresponding cyclohexadione and the lithium reagent by synthetic methods well known to a skilled man. The cyclohexadione may be obtained commercially.

The foregoing process typically produces a compound of formula (I) having trans geometry at the 1,3-, 1,4-, or 1,5-positions of the cycloalkane ring. Thus reduction of the ketone produces the cis alcohol which is converted in turn to the trans bis-BOC compound, the trans hydroxylamine (II) and the trans compound of formula (I).

To obtain the cis compound of formula (I), the cis alcohol is esterified using the appropriate carboxylic acid, typically in the presence of triphenylphosphine and diethylazodicarboxylate at low temperature in a non-polar solvent such as THF, to give the trans ester which is then hydrolysed using, for example, sodium bicarbonate in a polar solvent such as ethanol, to give the trans alcohol. This is converted in turn to the cis bis-BOC compound, the cis hydroxylamine (II) and the cis compound of formula (I).

Compounds of formula (I) wherein $R^1$ is an unsubstituted amino group, ie $R^2 = R^3 =$ hydrogen, may be converted to a corresponding compound of formula (I) wherein $R^1$ is a monosubstituted amino group, ie $R^2$ is hydrogen and $R^3$ is as hereinbefore defined, by treatment with the appropriate isocyanate ie $R^3NCO$, in a non-polar solvent, such as THF, and to a corresponding compound of formula (I) wherein $R^1$ is a disubstituted amino group, ie $R^2$ and $R^3$, which may be the same or different, are as hereinbefore defined, by treatment with phosgene or an equivalent thereof, for example, 1,1'-carbonyldiimidazole, followed by reaction in situ with the appropriate secondary amine, ie $HNR^2R^3$, in a non-polar solvent, such as THF or dichloromethane.

In the case where n = p = 1 or 2 and W is $-N<$ , the heterocyclic ketone may be prepared by reacting a compound of formula (III)

$Ar-(CH_2)_m-NH_2$ (III)

where Ar and m are as hereinbefore defined, with a compound of formula (IV)

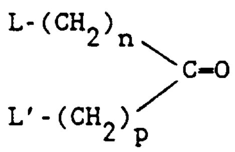

(IV)

wherein n and p are as hereinbefore defined, L and L', which may be the same or different, are suitable leaving groups, for example, alkanesulphonyloxy, and the carbonyl group is preferably protected, for example, as the dioxolane, typically by heating in 1,3-dimethyl-3,4,5,6-tetrahydro-2(1H)-pyrimidinone (DMPU) in the presence of sodium bicarbonate. The carbonyl group is then deprotected, for example, in the case where the protecting group is dioxolane, by treatment with dil. acid.

According to another aspect of the invention, there is provided a process for preparing compounds of formula (I) wherein m, Ar, R, W and Z are as hereinbefore defined, n = p = 1 or 2, and $R^1$ is $C_{1-6}$ alkyl, which comprises reacting a compound of formula (II) as hereinbefore defined with a suitable agent or agents and under such reaction conditions as to effect conversion of the N-hydrogen to an $N-C(Z)R^1$ group wherein $R^1$ is $C_{1-6}$ alkyl. According to whether Z is to be oxygen or sulphur, the reaction is typically carried out by treatment of the compound of formula (II) with at least two equivalents of an appropriate (thio)-alkanoylating agent, typically the sym. (thio)anhydride or (thio)acid halide, to give the N,O-bis-(thio)-alkanoylated compound, which compound is then partially hydrolysed, typically using anhy. potassium carbonate in a polar solvent, such as methanol.

Optional conversion of a compound of formula (I) to a corresponding base salt may conveniently be effected by reaction with the appropriate base.

For a better understanding of the invention, the following Examples are given by way of illustration.

SYNTHETIC EXAMPLES

Synthetic Example 1

Preparation of cis-1-[3-[3-(4-fluorophenoxy)phenyl]cyclobutyl}-1-hydroxyurea

(a) 1-Bromo-3-(4-fluorouhenoxy)benzene

An aqueous solution of KOH (49.0g in 100ml water) was added to a stirred solution of 4-fluorophenol (84.0g, Aldrich) in methanol (250ml). When additon was complete, the mixture was evaporated in vacuo and the residual solid pulverised and taken up in 1-methyl-2-pyrrolidinone (300ml). 3-Fluorobromobenzene (Aldrich, 131.3g), was added and the mixture refluxed for 24 hours, then cooled, poured into water (1500ml) and extracted with ether. The combined extracts were washed with 2M aqu. NaOH, 2M aqu. HCl and water, dried and evaporated in vacuo. The remaining oil was distilled to give the desired product (103.0g) at bp 85-100 °C/0.25 mm Hg. $^1$H NMR consistent with proposed structure.

(b) 3-(4-Fluorophenoxy)benzaldehyde)

A solution of butyl lithium in hexane (Fluka, 1.6M, 175ml) was added dropwise to a stirred solution of the product from step (a) at -65 °C. When addition was complete, the mixture was stirred at -70 °C for 10 minutes. A solution of dimethylformamide (DMF) (29.3g) in THF (50ml) was then added dropwise at -65 °C. When addition was complete, the mixture was stirred at -65 °C for 30 minutes and then allowed to warm to 0 °C. 2M Aqu. HCl (450ml) was added and the mixture stirred at room temperature for 15 minutes, then extracted with ether. The combined extracts were washed with water, dried and evaporated in vacuo to give an orange oil which was flash chromatographed through a silica column using hexane: ether (5:1) as eluant to give the desired product as a colourless oil (49.5g) which crystallised on standing, mp 47-48 °C. $^1$H NMR and 1R spectra consistent with proposed structure.

(c) 3-(4-Fluorophenoxy)phenylethene

Potassium t-butoxide (Aldrich, 3.4g) was added to a stirred suspension of methyltriphenylphosphonium bromide (Aldrich, 10.7g) in THF (100ml). When addition was complete, the mixture was stirred for 15 minutes until yellow and a solution of the product from step (b) (5.4g) in THF (25ml) added dropwise. When addition was complete, the mixture was stirred for 1.5 hours, quenched with water (150ml) and extracted with ether. The combined extracts were washed with water and satd. aqu. NaCl, dried and evaporated in vacuo to give an oily solid which was flash chromatographed through a silica column using dichloromethane as eluant to give the desired product as a pale yellow oil (5.4g). $^1$H NMR consistent with proposed structure.

(d) 2,2-Dichloro-3-[3-(4-fluorophenoxy)phenyl]cyclobutanone

A solution of trichloroacetyl chloride (Aldrich, 6.8g) and phosphoryl chloride (Aldrich, 5.8g) in ether (30ml) was added dropwise to a stirred mixture of the product from step (c) (5.4g) and copper-activated zinc powder (J.Org.Chem. 43, 2881 (1978), 2.6g) in ether (60ml). When addition was complete, the mixture was refluxed for 2 hours, cooled and further activated zinc (2.6g) added. The mixture was stirred overnight at room temperature and a solution of the acid chloride (1.8g) and phosphoryl chloride (1.5g) in ether (10ml) then added dropwise. When addition was complete, the mixture was refluxed for 1.5 hours, cooled and filtered through Hyflo. The filtrate was quenched with ice/water (150ml) and the mixture stirred for 15 minutes. The organic layer was separated, washed with satd. aqu. $NaHCO_3$, water and satd. aqu. NaCl, dried, treated with charcoal, filtered through Hyflo and evaporated in vacuo to give the desired product as a pale yellow oil (7.1g).

(e) 3-[3-(4-Fluorophenoxy)phenyl]cyclobutanone

A solution of the product from step (d) (7.1g) in glac. acetic acid (10ml) was added dropwise to a vigorously stirred mixture of zinc powder (BDH, 11.6g) in glac. acetic acid (18ml). When addition was complete, the mixture was stirred at 100 °C for 3 hours, cooled and poured into ice/water (300ml). The mixture was adjusted to pH 6 using 10M aqu. NaOH and extracted with ether. The combined extracts were washed with satd. aqu. $NaHCO_3$ and water, dried, treated with charcoal, filtered through Hyflo and

evaporated in vacuo to give the desired product as a pale yellow oil (5.6g). $^1$H NMR consistent with proposed structure.

(f) cis-3-[3-(4-Fluorophenoxy)phenyl]cyclobutanol

A solution of the product from step (e) (4.7g) in THF (25ml) was added dropwise to a stirred suspension of lithium aluminium hydride (Aldrich, 0.52g) in THF (75ml), the temperature of the mixture not exceeding 20 °C. When addition was complete, the mixture was stirred for 4 hours at room temperature, cooled in ice/water and cautiously decomposed using water (10ml). Further water (50ml) was added and the organic layer separated, washed with 1M aqu. HCl and satd. aqu. NaCl, dried and evaporated in vacuo to give the desired product as a pale yellow oil (4.6g). $^1$H NMR consistent with proposed structure.

(g) trans-3-[3-(4-Fluorophenoxy)phenyl]cyclobutyl acetate

A solution of diethylazodicarboxylate (Aldrich, 6.1g) in THF (30ml) was added dropwise to a stirred mixture of the product from step (f) (4.6g), triphenylphosphine (BDH, 9.2g) and glac. acetic acid (1.3g) in THF (50ml) at -5 °C. When addition was complete, the mixture was stirred at -5 to 0 °C for 2 hours. Further dicarboxylate (3.1g), triphenylphosphine (4.6g) and glac. acetic acid (0.7g) were added and the mixture stirred at room temperature for 2 hours, then evaporated in vacuo. The residue was flash chromatographed twice through a silica column using dichloromethane and ether: 40-60 pet. ether (1:4) respectively as eluants to give the desired product as a colourless oil (2.8g). $^1$H NMR consistent with proposed structure.

(h) trans-3-[3-(4-Fluorophenoxy)phenyl]cyclobutanol

Anhy. potassium carbonate (2.6g) followed by water (10ml) was added to a stirred solution of the product from step (g) (2.8g) in ethanol (40ml). When addition was complete, the mixture was stirred at 50 °C for 4 hours, cooled and evaporated in vacuo. The residue was partitioned between water (50ml) and ether (200ml) and the organic layer separated, washed with water, dried and evaporated in vacuo to give the desired product as a colourless oil (2.4g). $^1$H NMR consistent with proposed structure.

(i) cis-N,O-Bis(t-butoxycarbonyl)-N-{3-[3-(4-fluorophenoxy)phenyl] cyclobutyl}hydroxylamine

A solution of diethyl azodicarboxylate (DEAD) (Aldrich, 2.4g) in THF (10ml) was added dropwise to a stirred solution of the product from step (h) (2.4g), N,O-bis(t-butoxycarbonyl)hydroxylamine (BDH, 2.2g) and triphenylphosphine (Aldrich, 3.6g) in THF (45ml) at -5 °C. When addition was complete, the mixture was stirred at -5 to 0 °C for 5 hours. Further DEAD (1.6g in THF (10ml)) and triphenylphosphine (2.4g) were added and the mixture stirred at -5 °C for 2 hours, then overnight at room temperature and evaporated in vacuo. The residue was flash chromatographed twice through a silica column using dichloromethane and ether:40-60 pet. ether (1:4) respectively as eluants to give the desired product as a colourless oil (3.6g).

(j) cis-1-{3-[3-(4-Fluorophenoxy)phenyl]cyclobutyl}hydroxylamine

A solution of the product from step (i) (3.6g) and c.HCl (3.7ml) in methanol (50ml) was refluxed for 1 hour, cooled and evaporated in vacuo. The residual oil was taken up in water (25ml), carefully basified with satd. aqu. $NaHCO_3$ and extracted with ether. The combined extracts were dried and evaporated in vacuo to give the desired product as a colourless oil (2.2g).

(k) cis-1-{3-[3-(4-Fluorophenoxy)phenyl]cyclobutyl}-1-hydroxyurea

Potassium cyanate (BDH, 1.9g) followed by 2M aqu. HCl (7.7ml) was added to a stirred solution of the product from step (j) (2.1g) in THF (50ml) at 0 to 4 °C. When addition was complete, the mixture was stirred at 4 °C for 1.5 hours, then diluted with ether (50ml) and ethyl acetate (100ml), washed with water and satd. aqu. NaCl and evaporated in vacuo. The residue was recrystallised from EtOAc:$Et_2O$:40-60 pet. ether to give the desired product as a colourless solid (1.7g), mp 150-152 °C.
Anaylsis: C 64.43% (64.54%); H 5.42% (5.42%); N 8.74% (8.86%)
IR $\nu_{max}$ (KBr): 3466, 3261, 1645, 1502 and 1200 $cm^{-1}$
$^1$H NMR (200 MHz, δ, DMSO-$d_6$): 9.16 (1H, s, OH); 7.25, 7.08 and 6.85 (8H, m, ArH), 6.27 (2H, s, $NH_2$), 4.64 (1H, m, ArCH), 3.07 (1H, m, NCH), 2.35 (4H, m, cyclomethylene).

Synthetic Example 2

Preparation of trans-1-[3-(3-phenoxyphenyl)cyclobutyl]-1-hydroxyurea

(a) (3-Phenoxyphenyl)ethene

Potassium t-butoxide (Aldrich, 3.7g) was added to a stirred suspension of methyltriphenylphosphonium bromide (Aldrich, 10.7g) in THF (100ml). When addition was complete, the mixture was stirred for 15 minutes until yellow. Sufficient 3-phenoxybenzaldehyde (Aldrich) was added dropwise to the mixture to discharge the colour, then further phosphonium salt and/or potassium t-butoxide was added to restore the colour, followed by further 3-phenoxybenzaldehyde to discharge same. This process was repeated until all of the 3-phenoxybenzaldehyde (5.9g) had been consumed. When addition was complete, the mixture was stirred for 2.5 hours, then diluted with ether and filtered through Hyflo. The filtrate was washed with 2M aqu. HCl, satd. aqu. $NaHCO_3$, water and satd. aqu. NaCl, dried and evaporated in vacuo. The residue was flash chromatographed through a silica column using dichloromethane as eluant to give the desired product as a colourless oil (5.5g). $^1$H NMR consistent with proposed structure.

(b) 2,2-Dichloro-3-(3-phenoxyphenyl)cyclobutanone

A solution of trichloroacetyl chloride (Aldrich, 15.9g) and phosphoryl chloride (Aldrich, 13.4g) in ether (50ml) was added dropwise over 1 hour to a stirred mixture of the product from step (a) (5.5g) and copper-activated zinc powder (J.Org.Chem 43, 2881 (1978), 6.2g) in ether (80ml). When addition was complete, the mixture was refluxed for 2 hours, then cooled and filtered through Hyflo. The filtrate was partially evaporated in vacuo, diluted with pet. ether and cautiously treated with water until exothermicity ceased. The organic phase was separated and cautiously washed with satd. aqu. $NaHCO_3$ and satd. aqu. NaCl, then dried and filtered. The filtrate was evaporated in vacuo to give the desired product as a viscous yellow oil (8.6g). $^1$H NMR consistent with proposed structure.

(c) 3-(3-Phenoxyphenyl)cyclobutanone

A solution of the product from step (b) (8.6g) in glac. acetic acid (11ml) was added dropwise to a vigorously stirred mixture of copper-activated zinc powder (J.Org.Chem. 43, 2881 (1978), 14.6g) in glac. acetic acid (23ml). When addition was complete, the mixture was stirred at 70 ° C for 3 hours, then overnight at room temperature. The mixture was diluted with glac. acetic acid and ether, filtered through Hyflo and the filtrate diluted with water and extracted with ether. The organic phase was separated and cautiously washed with satd. aqu. $NaHCO_3$ and water, then dried and filtered. The filtrate was evaporated in vacuo and the residue flash chromatographed through a silica column using ether/pet. ether (1:1) as eluant to give the desired product as a colourless oil (5.6g). $^1$H NMR and IR consistent with proposed structure.

(d) cis-3-(3-Phenoxyphenyl)cyclobutanol

A solution of the product from step (c) (2.3g) in THF (20ml) was added dropwise to a stirred suspension of lithium aluminium hydride (Aldrich, 0.90g) in THF (100ml), the temperature of the mixture not exceeding 35 ° C. When addition was complete, the mixture was refluxed for 2 hours, cooled in ice/water and cautiously decomposed using 5M aqu. NaOH (25ml) and water (25ml). The organic layer was separated, washed with 2M aqu. HCl, water and satd. aqu. NaCl, dried and filtered. The filtrate was evaporated in vacuo to give the desired product as a pale greenish oil (5.4g). $^1$H NMR consistent with proposed structure.

(e) trans-N,O-bis(t-butoxycarbonyl)-N-[3-(3-phenoxyphenyl)cyclobutyl]hydroxylamine

A solution of diethyl azodicarboxylate (Aldrich, 5.2g) in THF (15ml) was added dropwise over 15 minutes to a stirred solution of the product from step (d) (3.6g) N,O-bis(t-butoxycarbonyl)hydroxylamine (BDH, 3.7g) and triphenylphosphine (Aldrich, 7.9g) in THF (100ml) at -5 ° C. When addition was complete, the mixture was stirred at -5 ° C for another 2 hours, then overnight at room temperature. The mixture was evaporated in vacuo and the residue flash chromatographed through a silica column using dichloromethane to give the desired product as a colourless oil (3.8g). $^1$H NMR consistent with proposed structure.

(f) trans-1-[3-(3-Phenoxyohenyl)cyclobutyl]hydroxylamine

A solution of the product from step (e) (3.8g) and c.HCl (3.9ml) in methanol (54ml) was refluxed for 1.5 hours, then evaporated in vacuo and the residue partitioned between ether and satd. aqu. $NaHCO_3$. The organic phase was separated, washed with water, dried and filtered. The filtrate was evaporated in vacuo to give the desired product as a colourless oil (1.9g). $^1H$ NMR consistent with proposed structure.

(g) trans-1-[3-(3-phenoxyphenyl)cyclobutyl]-1-hydroxyurea

2M aqu. HCl (15ml) was added dropwise over 30 minutes to a stirred solution of the product from step (f) (1.9g) and potassium cyanate (Aldrich, 1.9g) in THF (35ml) at 0°C. When addition was complete, the mixture was stirred at 0°C for an hour, then extracted with ether. The organic phase was washed with water and satd. aqu. NaCl, dried and filtered. The filtrate was evaporated in vacuo and the residue recrystallised from ethyl acetate to give the desired product as a fluffy, colourless solid (1.9g), mp 134-136°C.
Analysis: C 68.13% (calcd. 68.44%); H 6.06% (6.08%); N 9.31% (9.39%)
IR $\nu_{max}$ (KBr): 3491, 3153 and 1637 $cm^{-1}$
$^1H$ NMR (200MHz, $\delta$, DMSO-$d_6$): 9.35 (1H, s, OH), 7.38 & 7.00 (9H, m, ArH), 6.30 (2H, s, $NH_2$), 4.80 (1H, m, ArCH), 3.40 (1H, m, NCH), 2.70 & 2.18 (4H, m, cyclomethylene)

Synthetic Examples 3-23

The following compounds were prepared by methods analogous to those described in Synthetic Examples 1 and 2:

3) trans-1-{3-[3-(4-fluorophenoxy)phenyl]cyclobutyl}-1-hydroxyurea, mp 124-5°C;
4) cis-1-[3-(3-phenoxyphenyl)-1-cyclobutyl]-1-hydroxyurea, mp 134.5 -136.5°C;
5) trans-1-[3-(2-benzo[b]thienyl)cyclobutyl]-1-hydroxyurea, mp 165-6°C;
6) trans-1-[3-(3-phenoxyphenyl)cyclohexyl]-1-hydroxyurea, mp 156-8°C;
7) cis-1-[3-(3-phenoxyphenyl)cyclohexyl]-1-hydroxyurea, mp 125°C (dec.);
8) trans-1-{3-[3-(4-Fluorophenoxy)phenyl]cyclobutyl}acetohydroxamic acid, mp 107-8°C;
9) trans-1-{3-[3-(4-Fluorophenoxy)phenyl]-3-methylcyclobutyl}-1-hydroxyurea, mp 125-126°C;
10) cis-1-{3-[3-(4-Fluorophenoxy)phenyl]-3-methylcyclobutyl}-1-hydroxyurea, mp 140.5-141.5°C;
11) trans-1-[3-(3-Methoxyphenyl)cyclobutyl]-1-hydroxyurea. 0.15 hydrate, mp 123-124°C;
12) trans-1-[3-(4-Phenoxyphenyl)cyclobutyl]-1-hydroxyurea, mp 165-167°C;
13) cis-1-[3-(4-Phenoxyphenyl)cyclobutyl]-1-hydroxyurea, mp 172-173°C;
14) cis-1-[3-(3-Methoxyphenyl)cyclobutyl]-1-hydroxyurea. 0.17 hydrate, mp 145-147°C;
15) trans-1-[3-(4-Fluorophenyl)cyclobutyl]-1-hydroxyurea, mp 140.5-141.5°C;
16) cis-1-[3-(4-Fluorophenyl)cyclobutyl]-1-hydroxyurea, mp 145-147°C;
17) 1-[3-Butyl-3-(3-phenoxyphenyl)cyclobutyl]-1-hydroxyurea, off-white foam;
18) cis-1-{3-[3-(4-Fluorophenoxy)phenyl]cyclobutyl}-1-hydroxythiourea, mp 162-163°C;
19) 1-{1-[3-(4-Fluorophenoxy)phenyl]-4-piperidinyl}-1-hydroxyurea, mp 153-154°C (dec.);
20) trans-1-{3-[3-(4-Cyanophenoxy)phenyl]cyclobutyl}-1-hydroxyurea, mp 139-140°C;
21) cis-1-{3-[3-(4-Fluorophenoxy)phenyl]-1-carbamoyl-1-cyclobutyl}-1-hydroxyurea, mp 129-131°C;
22) 1-(3,3-Diphenylcyclobutyl)-1-hydroxyurea. 0.18 hydrate, colourless amorphous solid;
23) cis-1-{3-[3-(4-Fluorophenoxy)phenyl]cyclobutyl}-1-hydroxy-3-methylurea, mp 123-124°C; and
24) cis-1-[2-(3-Phenoxyphenyl)-1-cyclobutyl]-1-hydroxyurea.

The $^1H$ NMRs and elemental analyses of these compounds were consistent with the proposed structures.

PHARMACEUTICAL FORMULATION EXAMPLES

The "active ingredient" in the following formulations is any compound of formula (I) as hereinbefore defined, for example, any of the compounds of Synthetic Examples 1 to 24.

Example A: Tablet

| | Per tablet |
|---|---|
| Active Ingredient | 5.0 mg |
| Lactose | 82.0 mg |
| Starch | 10.0 mg |
| Povidone | 2.0 mg |
| Magnesium Stearate | 1.0 mg |

Mix together the active ingredient, lactose and starch. Granulate the powders using a solution of povidone in purified water. Dry the granules, add the magnesium stearate and compress to produce tablets (100mg per tablet).

Example B: Ointment

| | |
|---|---|
| Active Ingredient | 1.0 g |
| White Soft Paraffin to | 100.0 g |

Disperse the active ingredient in a small volume of the vehicle. Gradually incorporate this into the bulk to produce a smooth, homogeneous product. Fill into collapsible metal tubes.

Example C: Cream for topical use

| | |
|---|---|
| Active Ingredient | 1.0 g |
| Polawax GP 200 | 20.0 g |
| Lanolin Anhydrous | 2.0 g |
| White Beeswax | 2.5 g |
| Methyl hydroxybenzoate | 0.1 g |
| Distilled Water to | 100.0 g |

Heat the Polawax, beeswax and lanolin together at 60°C. Add a solution of methyl hydroxybenzoate. Homogenise using high speed stirring. Allow the temperature to fall to 50°C. Add and disperse the active ingredient. Allow to cool with slow speed stirring.

Example D: Lotion for topical use

| | |
|---|---|
| Active Ingredient | 1.0 g |
| Sorbitan Monolaurate | 0.6 g |
| Polysorbate 20 | 0.6 g |
| Cetostearyl Alcohol | 1.2 g |
| Glycerin | 6.0 g |
| Methyl Hydroxybenzoate | 0.2 g |
| Purified Water B.P. to | 100 ml |

The methyl hydroxybenzoate and glycerin were dissolved in 70ml of the water at 75°C. The sorbitan monolaurate, Polysorbate 20 and cetostearyl alcohol were melted together at 75°C and added to the aqueous solution. The resulting emulsion was homogenised, allowed to cool with continuous stirring and the active ingredient added as a suspension in the remaining water. The whole was stirred until homogeneous.

Example E: Eye drops

| | |
|---|---|
| Active Ingredient | 0.5 g |
| Methyl Hydroxybenzoate | 0.01 g |
| Propyl Hydroxybenzoate | 0.04 g |
| Purified Water B.P. to | 100 ml |

The methyl and propyl hydroxybenzoates were dissolved in 70ml of purified water at 75°C and the resulting solution allowed to cool. The active ingredient was then added and the solution made up to 100ml with purified water. The solution was sterilised by filtration through a membrane filter of 0.22µm pore size and packed aseptically into suitable sterile containers.

Example F: Injectable solution

| | |
|---|---|
| Active Ingredient | 10.0 mg |
| Water for Injections B.P. to | 1.0 ml |

The active ingredient was dissolved in half of the Water for Injections and then made up to volume and sterilised by filtration. The resulting solution was distributed into ampoules under aseptic conditions.

Example C: Powder capsules for inhalation

| | |
|---|---|
| Active Ingredient (0.5-7.0µm powder) | 4 mg |
| Lactose (30-90µm powder) | 46.0 mg |

The powders were mixed until homogeneous and filled into suitably sized hard gelatin capsules (50mg per capsule).

Example H: Inhalation aerosol

| | |
|---|---|
| Active Ingredient (0.5-7.0µm powder) | 200 mg |
| Sorbitan Trioleate | 100 mg |
| Saccharin Sodium (0.5-7.0µm powder) | 5 mg |
| Methanol | 2 mg |
| Trichlorofluoromethane | 4.2 g |
| Dichlorodifluoromethane to | 10.0 ml |

The sorbitan trioleate and menthol were dissolved in the trichlorofluoromethane. The saccharin sodium and active ingredient were dispersed in the mixture which was then transferred to a suitable aerosol canister and the dichlorofluoromethane injected through the valve system. This composition provides 2mg of active ingredient in each 100µl dose.

BIOLOGICAL DATA

In vitro inhibition of 5-lipoxygenase

Neutrophils were isolated from whole blood taken from aspirin-free volunteers. The cells were suspended in HEPES/Hanks buffer (pH 7.4) to give a working concentration of 1 x $10^6$ cells/ml. A solution of the test compound in DMSO (10µl, final concentration in the range 0.001 - 10µM) was added to the washed neutrophils (470µl) and allowed to equilibrate at room temperature for 5 minutes. The tubes were placed on

ice and a solution of the calcium ionophore A23187 (20µl of 50µM stock, final concentration 2µM) was added to the tubes which were then incubated at 37°C for 5 minutes. The reaction was stopped by boiling. Radioimmunoassay was conducted for leukotriene $B_4$.

For the compounds of Synthetic Examples 1 and 2, the results were as follows:

| Synthetic Example | $IC_{50}$(µM) |
|---|---|
| 1 | 0.15 |
| 2 | 0.04 |

## Claims

**1.** A compound of formula (I)

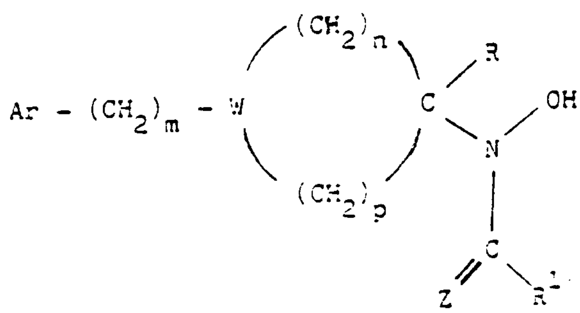

(I)

wherein

m is O or 1;

n is an integer of from 1 to 6 and p is an integer of from 0 to 3 with the proviso that n + p does not exceed 6;

Ar is a mono-, bi-, or tricyclic aromatic ring system wherein one or more of the carbon atoms is optionally replaced by a heteroatom(s) independently selected from oxygen, sulphur and nitrogen, which ring system is optionally substituted by one or more atoms or groups independently selected from (i) $C_{1-4}$ alkyl wherein one or more of the hydrogen atoms may be replaced by halogen, $C_{1-4}$ alkoxy, halogen, nitro, amino, carboxy, $C_{2-5}$ alkoxycarbonyl, hydroxy, cyano, carbamoyl and alkylsulphonyl, and (ii) phenoxy, benzyloxy and naphthyloxy, which aryloxy groups are optionally substituted by one or more substituents independently selected from those specified in (i);

R is hydrogen, $C_{1-6}$ alkyl, vinyl, $C_{1-6}$ alkoxy($C_{1-6}$ alkyl), or carbamoyl;

$R^1$ is $C_{1-6}$ alkyl (including $C_{3-6}$ cycloalkyl), phenyl, or benzyl, which phenyl or benzyl group is optionally substituted by one or more substituents independently selected from those specified in (i), or $-NR^2R^3$ wherein $R^2$ and $R^3$ are independently selected from hydrogen, $C_{1-6}$ alkyl (including $C_{3-6}$ cycloalkyl), phenyl and benzyl, which phenyl and benzyl groups are optionally substituted by one or more substituents independently selected from those specified in (i);

W is $-N<$ or $-C(R^4)<$ where $R^4$ is hydrogen, $C_{1-4}$ alkyl, or phenyl;

Z is oxygen or sulphur;

with the provisoes that

(i) $n + p \neq 1$ when W is $-C(R^4)<$ ; or

(ii) $n + p \neq 4$ when Ar is an unsubstituted phenyl group;

and base salts and physiologically functional derivatives thereof.

**2.** A compound of formula (I) as claimed in Claim 1, wherein

m is 0;

n = p = 1 or 2;

Ar is a substituted phenyl or an optionally substituted benzothienyl group;
R is hydrogen;
$R^1$ is $C_{1-4}$ alkyl or $-NR^2R^3$ wherein $R^2$ and $R^3$ are both hydrogen;
W is -CH< ; and
Z is oxygen;
and base salts and physiologically functional derivatives thereof.

**3.** A compound of formula (I) as claimed in Claim 1, which compound is cis-1-{3-[3-(4-fluorophenoxy)-phenyl]cyclobutyl}-1-hydroxyurea, trans-1-hydroxy-1-[3-(2-benzo[b]thienyl)cyclobutyl]urea, or a base salt or physiologically functional derivative of either thereof.

**4.** A compound of formula (I) as claimed in any of Claims 1 to 3, or a base salt or physiologically functional derivative thereof, for use as a therapeutic agent.

**5.** A compound of formula (I) as claimed in any of Claims 1 to 3, or a base salt or physiologically functional derivative thereof, for use in the prophylaxis or treatment of a clinical condition for which a 5-lipoxygenase inhibitor is indicated.

**6.** A compound of formula (I) as claimed in any of Claims 1 to 3, or a base salt or physiologically functional derivative thereof, for use in the prophylaxis or treatment of a clinical condition selected from spasmogenic conditions, allergic conditions, tumour formation, conditions involving blood platelet aggregation, and inflammatory conditions.

**7.** A compound of formula (I) as claimed in any of Claims 1 to 3, or a base salt or physiologically functional derivative thereof, for use in the prophylaxis or treatment of intrinsic (spasmogenic) or extrinsic (allergic) asthma or irritable (spasmogenic) bowel syndrome.

**8.** Use of a compound of formula (I) as claimed in any of Claims 1 to 3, or a base salt or physiologically functional derivative thereof, in the manufacture of a medicament for the prophylaxis or treatment of a clinical condition for which a 5-lipoxygenase inhibitor is indicated.

**9.** Use of a compound of formula (I) as claimed in any of Claims 1 to 3, or a base salt or physiologically functional derivative thereof, in the manufacture of a medicament for the prophylaxis or treatment of a clinical condition selected from spasmogenic conditions, allergic conditions, tumour formation, conditions involving blood platelet aggregation, and inflammatory conditions.

**10.** Use of a compound of formula (I) as claimed in any of Claims 1 to 3, or a base salt or physiologically functional derivative thereof, in the manufacture of a medicament for the prophylaxis or treatment of intrinsic (spasmogenic) or extrinsic (allergic) asthma or irritable (spasmogenic) bowel syndrome.

**11.** A medicament comprising a compound of formula (I) as claimed in any of Claims 1 to 3, or a base salt or physiologically functional derivative thereof, a pharmaceutically acceptable carrier and, optionally, one or more other physiologically active agents for use in the prophylaxis or treatment of a clinical condition for which a 5-lipoxygenase inhibitor is indicated.

**12.** A medicament as claimed in Claim 11 for use in the prophylaxis or treatment of a clinical condition selected from spasmogenic conditions, allergic conditions, tumour formation, conditions involving blood platelet aggregation, and inflammatory conditions.

**13.** A medicament as claimed in Claim 11 for use in the prophylaxis or treatment of intrinsic (spasmogenic) or extrinsic (allergic) asthma or irritable (spasmogenic) bowel syndrome.

**14.** A medicament as claimed in any of Claims 11 to 13 which is in the form of a tablet or capsule.

**15.** A process for the preparation of a compound of formula (I) as claimed in Claim 1 which comprises reacting a compound of formula (II)

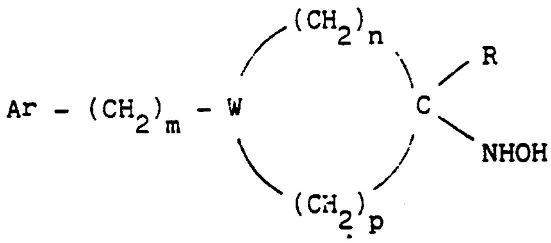

(II)

wherein m, n, p, Ar, R and W are as defined in Claim 1, with a suitable agent(s) and under such reaction conditions as to effect conversion of the N-hydrogen to an N-C(Z)$R^1$ group wherein $R^1$ and Z are as defined in Claim 1,
and optionally converting the compound of formula (I) so obtained to a base salt or physiologically functional derivative.

**16.** A method for the preparation of a medicament as claimed in any of Claims 11 to 13, which comprises
(a) preparing a compound of formula (I) or a base salt or physiologically functional derivative thereof by a process as claimed in Claim 15; and
(b) admixing the product from step (a) with a pharmaceutically acceptable carrier and, optionally, one or more other physiologically active agents.

**17.** A method as claimed in Claim 16 which comprises an additional step (c) wherein the admixture from step (b) is formed into a tablet or capsule.

**Patentansprüche**

**1.** Verbindung der Formel (I)

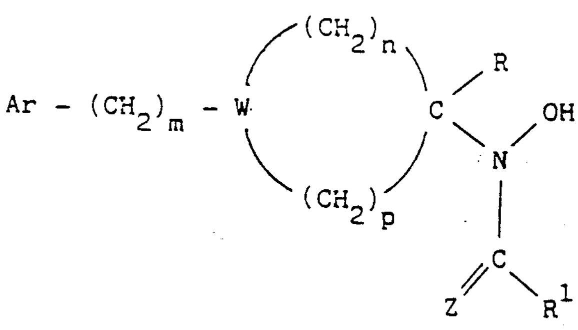

(I)

worin bedeuten:
m 0 oder 1;
n eine ganze Zahl von 1 bis 6 und p eine ganze Zahlvon 0 bis 3, mit der Maßgabe, daß n + p 6 nicht überschreitet;
Ar ein mono-, bi- oder tricyclisches aromatische Ringsystem, worin ein oder mehrere der Kohlenstoffatome wahlweise durch Heteroatom(e), welche unabhängig voneinander aus Sauerstoff, Schwefel und Stickstoffgewählt sind, substituiert sind, welches Ringsystem wahlweise durch ein oder mehrere Atome oder Gruppen substituiert ist, welche unabhängig voneinander aus (i) $C_{1-4}$-Alkyl, worin ein oder mehrere der Wasserstoffatome durch Halogen ersetzt sein können, $C_{1-4}$-Alkoxy, Halogen, Nitro, Amino, Carboxy, $C_{2-5}$-Alkoxycarbonyl, Hydroxy, Cyano, Carbamoyl und Alkylsulfonyl, und (ii) Phenoxy, Benzyloxy und Naphthyloxy, welche Aryloxygruppen wahlweise durch einen oder mehrere, unabhängig voneinander aus den in (i) genannten Substituenten gewfählte, substituiert sind, gewählt sind;
R Wasserstoff, $C_{1-6}$-Alkyl, Vinyl, $C_{1-6}$-Alkoxy($C_{1-6}$-alkyl) oder Carbamoyl;
$R^1$ $C_{1-6}$-Alkyl (einschließlich $C_{3-6}$-Cycloalkyl), Phenyl oder Benzyl, welche Phenyl- oder Benzylgruppe wahlweise durch einen oder mehrere Substituenten, welche unabhängig voneinander aus den in (i) beschriebenen gewählt sind, substituiert ist, oder -$NR^2R^3$, worin $R^2$ und $R^3$ unabhängig voneinander aus Wasserstoff, $C_{1-6}$-Alkyl (einschließlich $C_{3-6}$-Cycloalkyl), Phenyl und Benzyl gewählt sind, welche

Phenyl- und Benzylgruppen wahlweise durch einen oder mehrere Substituenten substituiert sind, welche unabhängig voneinander aus den in (i) beschriebenen gewählt sind;
W -N< oder -C($R^4$)< worin $R^4$ Wasserstoff, $C_{1-4}$-Alkyl oder Phenyl ist:
Z Sauerstoff oder Schwefel;
mit den Maßgaben, daß
(i) $n + p \neq 1$, wenn W die Bedeutung -C($R^4$)< hat; oder
(ii) $n + p \neq 4$, wenn Ar eine unsubstituierte Phenylgruppe ist:
und Basensalze, sowie physiologisch funktionelle Derivate davon.

2. Verbindung der Formel (I) nach Anspruch 1, worin bedeuten
m 0;
$n = p = 1$ oder 2;
Ar eine substituierte Phenyl- oder eine wahlweise substituierte Benzothienylgruppe;
R Wasserstoff;
$R^1$ $C_{1-4}$-Alkyl oder -$NR^2R^3$, worin $R^2$ und $R^3$ beide Wasserstoff sind;
W -CH< ; und
Z Sauerstoff;
und Basensalze sowie physiologisch funktionelle Derivate davon.

3. Verbindung der Formel (I) nach Anspruch 1, nämlich cis-1-{3-[3-(4-Fluorphenoxy)phenyl]cyclobutyl}-1-hydroxyharnstoff, trans-1-Hydroxy-1-[3-(2-benzo[b]thienyl)cyclobutyl]harnstoff oder ein Basensalz oder physiologisch funktionelles Derivat jeweils hiervon.

4. Verbindung der Formel (I) nach mindestens einem der Ansprüche 1 bis 3 oder ein Basensalz oder physiologisch funktionelles Derivat davon zur Verwendung als therapeutisches Mittel.

5. Verbindung der Formel (I) nach mindestens einem der Ansprüche 1 bis 3 oder ein Basensalz oder physiologisch funktionelles Derivat davon zur Verwendung bei der Prophylaxe oder Behandlung eines klinischen Zustands, für welchen ein 5-Lipoxygenaseinhibitor angezeigt ist.

6. Verbindung der Formel (I) nach mindestens einem der Ansprüche 1 bis 3 oder ein Basensalz oder physiologisch funktionelles Derivat davon zur Verwendung bei der Prophylaxe oder Behandlung eines klinischen Zustands, gewählt aus spasmogenen Zuständen, allergischen Zuständen, Tumorbildung, Zuständen, bei denen eine Blutplättchenaggregation involviert ist, und Entzündungszuständen.

7. Verbindung der Formel (I) nach mindestens einem der Ansprüche 1 bis 3 oder ein Basensalz oder physiologisch funktionelles Derivat davon zur Verwendung bei der Prophylaxe oder Behandlung von endogenem (spasmogenem) oder exogenem (allergischem) Asthma oder erregbarem (spasmogenem) Bowel-Syndrom.

8. Verwendung einer Verbindung der Formel (I) nach mindestens einem der Ansprüche 1 bis 3 oder eines Basensalzes oder physiologisch funktionellen Derivats davon bei der Herstellung eines Arzneimittels für die Prophylaxe oder Behandlung eines klinischen Zustands, bei welchem ein 5-Lipoxygenaseinhibitor angezeigt ist.

9. Verwendung einer Verbindung der Formel (I) nach mindestens einem der Ansprüche 1 bis 3 oder eines Basensalzes oder physiologisch funktionellen Derivats davon bei der Herstellung eines Arzneimittels für die Prophylaxe oder Behandlung eines klinischen Zustands, gewählt aus spasmogenen Zuständen, allergischen Zuständen, Tumorbildung, Zuständen, bei denen eine Blutplättchenaggregation involviert ist, und Entzündungszuständen.

10. Verwendung einer Verbindung der Formel (I) nach mindestens einem der Ansprüche 1 bis 3 oder eines Basensalzes oder physiologisch funktionellen Derivats davon bei der Herstellung eines Arzneimittels für die Prophylaxe oder Behandlung von endogenem (spasmogenem) oder exogenem (allergischem) Asthma oder erregbarem (spasmogenem) Bowel-Syndrom.

11. Arzneimittel, umfassend eine Verbindung der Formel (I) nach mindestens einem der Ansprüche 1 bis 3 oder ein Basensalz oder physiologisch funktionelles Derivat davon, einen pharmazeutisch annehmbaren

Träger und wahlweise ein oder mehrere andere physiologisch aktive Mittel zur Verwendung bei der Prophylaxe oder Behandlung eines klinischen Zustands, für welchen ein 5-Lipoxygenaseinhibitor angezeigt ist.

**12.** Arzneimittel nach Anspruch 11 zur Verwendung bei der Prophylaxe oder Behandlung eines klinischen Zustands, gewählt aus spasmogenen Zuständen, allergischen Zuständen, Tumorbildung, Zuständen, bei denen eine Blutplättchenaggregation involviert ist, und Entzündungszuständen.

**13.** Arzneimittel nach Anspruch 11 zur Verwendung bei der Prophylaxe oder Behandlung von endogenem (spasmogenem) oder exogenem (allergischem) Asthma oder erregbarem (spasmogenem) Bowel-Syndrom.

**14.** Arzneimittel nach mindestens einem der Ansprüche 11 bis 13 in Form einer Tabelle oder Kapsel.

**15.** Verfahren zur Herstellung einer Verbindung der Formel (I) nach Anspruch 1, umfassend das Umsetzen einer Verbindung der Formel (II)

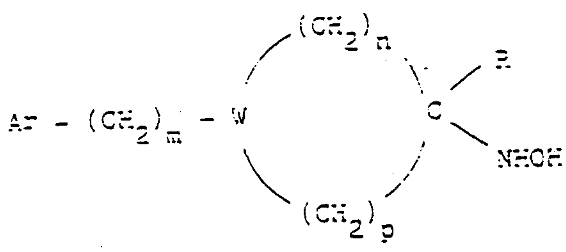

(II)

worin m, n, p, Ar, R und W wie in Anspruch 1 definiert sind, mit einem geeigneten Mittel(n) unter solchen Reaktionsbedingungen, um eine Umwandlung des N-Wasserstoffs in eine N-C(Z)$R^1$-Gruppe zu bewirken, worin $R^1$ und Z wie in Anspruch 1 definiert sind,
und wahlweise Umwandeln der so erhaltenen Verbindung der Formel (I) in ein Basensalz oder physiologisch funktionelles Derivat.

**16.** Verfahren zur Herstellung eines Arzneimittels nach mindestens einem der Ansprüche 11 bis 13, umfassend
(a) Herstellen einer Verbindung der Formel (I) oder eines Basensalzes oder physiologisch funktionellen Derivats davon durch ein Verfahren gemäß Anspruch 15; und
(b) Vermischen des Produkts aus Schritt (a) mit einem pharmazeutisch annehmbaren Träger und wahlweise einem oder mehreren anderen physiologisch aktiven Mitteln.

**17.** Verfahren nach Anspruch 16, umfassend einen zusätzlichen Schritt (c), worin die Mischung aus Schritt (b) zu einer Tablette oder Kapsel geformt wird.

## Revendications

**1.** Composé de formule (I)

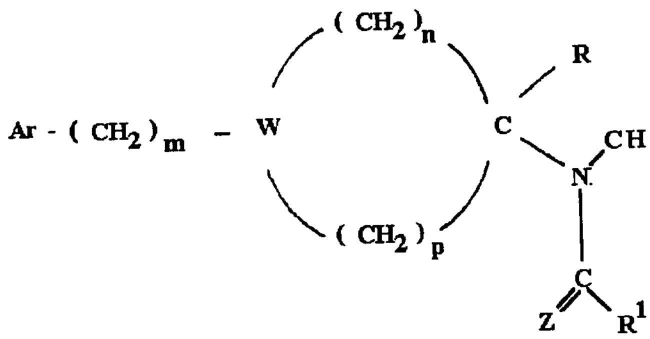

(I)

dans laquelle

- m est 0 ou 1 ;
- n est un nombre entier de 1 à 6 et p est un nombre entier de 0 à 3, à condition que n + p ne soit pas supérieur à 6 ;
- Ar représente un système cyclique aromatique mono-, bi-, ou tricyclique, dans lequel un ou plusieurs atomes de carbone sont éventuellement remplacés par un (des) hétéroatome(s) choisi(s) indépendamment parmi l'oxygène, le soufre et l'azote, système cyclique qui est éventuellement substitué par un ou plusieurs atomes ou groupes, choisis indépendamment parmi (i) un groupe alkyle en $C_{1-4}$ dans lequel un ou plusieurs atomes d'hydrogène peuvent être remplacés par un atome d'halogène, un groupe alkoxy en $C_{1-4}$, un atome d'halogène, un groupe nitro, amino, carboxy, alkoxycarbonyle en $C_{2-5}$, hydroxy, cyano, carbamoyle et un groupe alkylsulfonyle, et (ii) un groupe phénoxy, benzyloxy et un groupe naphtyloxy, lesdits groupes aryloxy étant éventuellement substitués par un ou plusieurs substituants choisis indépendamment parmi ceux spécifiés en (i) ;
- R représente un atome d'hydrogène, un groupe alkyle en $C_{1-6}$, vinyle, alkoxy en $C_{1-6}$ (alkyle en $C_{1-6}$) ou un groupe carbamoyle ;
- $R^1$ représente un groupe alkyle en $C_{1-6}$ (y compris cycloalkyle en $C_{3-6}$), phényle ou un groupe benzyle, ledit groupe phényle ou benzyle étant éventuellement substitué par un ou plusieurs substituants choisis indépendamment parmi ceux spécifiés en (i), ou un groupe $-NR^2R^3$ dans lequel $R^2$ et $R^3$ sont choisis indépendamment parmi un atome d'hydrogène, un groupe alkyle en $C_{1-6}$ (y compris cycloalkyle en $C_{3-6}$), phényle et un groupe benzyle, lesdits groupes phényle et benzyle étant éventuellement substitués par un ou plusieurs substituants choisis indépendamment parmi ceux spécifiés en (i) ;
- W représente $-N\langle$ ou $-C(R^4)\langle$ dans lequel $R^4$ représente un atome d'hydrogène, un groupe alkyle en $C_{1-4}$, ou un groupe phényle ;
- Z représente un atome d'oxygène ou un atome de soufre ;

à la condition que

(i) $n + p \neq 1$ lorsque W représente $-C(R^4)\langle$ ; ou

(ii) $n + p \neq 4$ lorsque Ar représente un groupe phényle non substitué ;

et sels d'une base et dérivés physiologiquement fonctionnels de celui-ci.

**2.** Composé de formule (I), tel que revendiqué dans la revendication 1, dans lequel

m est 0 ;

n = p = 1 ou 2 ;

- Ar représente un groupe phényle substitué ou un groupe benzothiényle éventuellement substitué ;
- R représente un atome d'hydrogène ;
- $R^1$ représente un groupe alkyle en $C_{1-4}$ ou un groupe $-NR^2R^3$ dans lequel $R^2$ et $R^3$ sont tous

deux des atomes d'hydrogène ;

W représente -CH< ; et

Z représente un atome d'oxygène ;

et sels d'une base et dérivés physiologiquement fonctionnels de celui-ci.

3. Composé de formule (I), tel que revendiqué dans la revendication 1, composé qui est la cis-1-(3-[3-(4-fluorophénoxy)phényl]cyclobutyl)-1-hydroxyurée, la trans-1-hydroxy-1-[3-(2-benzo[b]thiényl)cyclobutyl]-urée, ou un sel d'une base ou un dérivé physiologiquement fonctionnel de l'un quelconque de ceux-ci.

4. Composé de formule (I), tel que revendiqué dans l'une quelconque des revendications 1 à 3, ou un sel d'une base ou un dérivé physiologiquement fonctionnel de celui-ci, pour utilisation comme agent thérapeutique.

5. Composé de formule (I), tel que revendiqué dans l'une quelconque des revendications 1 à 3, ou un sel d'une base ou un dérivé physiologiquement fonctionnel de celui-ci, pour utilisation dans le traitement curatif ou préventif d'une affection clinique pour laquelle un inhibiteur de la 5-lipoxygénase est indiqué.

6. Composé de formule (I), tel que revendiqué dans l'une quelconque des revendications 1 à 3, ou un sel d'une base ou un dérivé physiologiquement fonctionnel de celui-ci, pour utilisation dans le traitement curatif ou préventif d'une affection clinique choisie parmi des affections spasmogènes, affections allergiques, formation de tumeur, affections faisant intervenir l'agrégation des plaquettes sanguines et affections inflammatoires.

7. Composé de formule (I), tel que revendiqué dans l'une quelconque des revendications 1 à 3, ou un sel d'une base ou un dérivé physiologiquement fonctionnel de celui-ci, pour utilisation dans un traitement curatif ou préventif de l'asthme intrinsèque (spasmogène) ou extrinsèque (allergique) ou du syndrome du colon irritable (spasmogène).

8. Utilisation d'un composé de formule (I), tel que revendiqué dans l'une quelconque des revendications 1 à 3, ou un sel d'une base ou un dérivé physiologiquement fonctionnel de celui-ci, dans la préparation d'un médicament pour le traitement curatif ou préventif d'une affection clinique dans laquelle un inhibiteur de la 5-lipoxygénase est indiqué.

9. Utilisation d'un composé de formule (I), tel que revendiqué dans l'une quelconque des revendications 1 à 3, ou un sel d'une base ou un dérivé physiologiquement fonctionnel de celui-ci, dans la préparation d'un médicament pour le traitement curatif ou préventif d'une affection clinique choisie parmi des affections spasmogènes, affections allergiques, formation de tumeur, affections faisant intervenir l'agrégation des plaquettes sanguines et affections inflammatoires.

10. Utilisation d'un composé de formule (I), tel que revendiqué dans l'une quelconque des revendications 1 à 3, ou un sel d'une base ou un dérivé physiologiquement fonctionnel de celui-ci, dans la préparation d'un médicament pour le traitement curatif ou préventif de l'asthme intrinsèque (spasmogène) ou extrinsèque (allergique) ou du syndrome du colon irritable (spasmogène).

11. Médicament comprenant un composé de formule (I), tel que revendiqué dans l'une quelconque des revendications 1 à 3 ou un sel d'une base ou un dérivé physiologiquement fonctionnel de celui-ci, un véhicule pharmaceutiquement acceptable et, éventuellement, un ou plusieurs agents physiologiquement actifs pour utilisation dans le traitement curatif ou préventif d'une affection clinique dans laquelle un inhibiteur de la 5-lipoxygénase est indiqué.

12. Médicament tel que revendiqué dans la revendication 11, pour utilisation dans le traitement curatif ou préventif d'une affection clinique choisie parmi des affections spasmogènes, affections allergiques, formation de tumeur, affections faisant intervenir l'agrégation des plaquettes sanguines et affections inflammatoires.

13. Médicament tel que revendiqué à la revendication 11, pour utilisation dans le traitement curatif ou préventif de l'asthme intrinsèque (spasmogène) ou extrinsèque (allergique) ou du syndrome du colon irritable (spasmogène).

**14.** Médicament tel que revendiqué dans l'une quelconque des revendications 11 à 13, qui se présente sous la forme de comprimé ou de capsule.

**15.** Procédé pour la préparation d'un composé de formule (I) tel que revendiqué dans la revendication 1, qui comprend l'étape consistant à faire réagir un composé de formule (II)

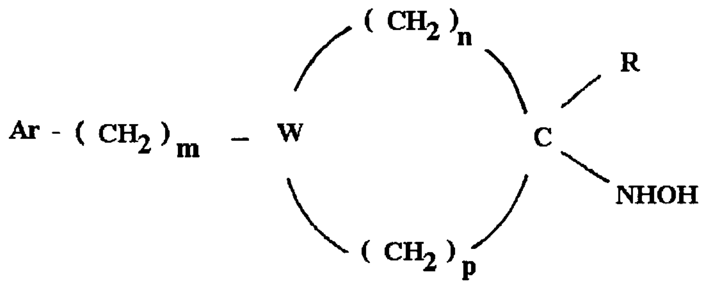

(II)

dans laquelle m, n, p, Ar, R et W sont tels que définis dans la revendication 1, avec un (des) agent(s) approprié(s) et dans des conditions réactionnelles entraînant la conversion du N-hydrogène en un groupe N-C(Z)$R^1$, dans lequel $R^1$ et Z sont tels que définis dans la revendication 1,
et éventuellement à convertir le composé de formule (I) ainsi obtenu en un sel d'une base ou en un dérivé physiologiquement fonctionnel.

**16.** Méthode pour la préparation d'un médicament tel que revendiqué dans l'une quelconque des revendications 11 à 13, qui comprend les étapes consistant à
(a) préparer un composé de formule (I) ou un sel d'une base ou un dérivé physiologiquement fonctionnel de celui-ci par un procédé tel que revendiqué à la revendication 15 ; et
(b) mélanger le produit de l'étape (a) avec un véhicule pharmaceutiquement acceptable et, éventuellement, un ou plusieurs autres agents physiologiquement actifs.

**17.** Méthode telle que revendiquée à la revendication 16, qui comprend une étape (c) supplémentaire dans laquelle le mélange de l'étape (b) est mis sous la forme d'un comprimé ou d'une capsule.